# EUROPEAN PATENT APPLICATION

(11) **EP 1 857 489 A1**
(43) Date of publication of application: **21.11.2007**
(21) Application number: 06728655.9
(22) Date of filing: 06.03.2006
(51) Int. Cl.: C08G 81/00, A61K 9/14, A61K 9/50, A61K 31/7105, A61K 31/711, A61K 38/00, A61K 47/32, A61K 47/34

(54) **MICROPARTICLE AND PHARMACEUTICAL COMPOSITION**

(30) Priority: 09.03.2005 JP 2005065619; 19.08.2005 JP 2005238846
(71) Applicant: TORAY INDUSTRIES, INC., Tokyo 103-8666 (JP)
(72) Inventor: KAKIZAWA, Yoshinori, Kamakura-shi, Kanagawa, 2480031 (JP); AOKI, Takao, Naka-gun, Kanagawa, 2590132 (JP); IDA, Nobuo, Kamakura-shi, Kanagawa, 2480033 (JP); NISHIO, Reiji, Fujisawa-shi, Kanagawa, 2510871 (JP)
(74) Representative: Kador & Partner
(86) International application number: PCT/JP2006/304236
(87) International publication number: WO 2006/095668

(57) **Abstract**

A fine particle comprising an amphiphilic polymer, further comprising an inner nucleus of hydrophilic segment of amphiphilic polymer, and a hydrophobic outer layer of hydrophobic segment of amphiphilic polymer, and having a surface modifier bonded to the hydrophobic outer layer.

A fine particle of the invention effectively enclose protein, peptide drug, nucleic acid medicine of hydrophilic property and large molecular weight in the inner nucleus of hydrophilic segment of amphiphilic polymer, and are preferable for stabilizing in the body and promoting absorption.

## Description

### TECHNICAL FIELD

The invention relates to a fine particle and a pharmaceutical preparation which effectively deliver bioactive substances, drugs, contrast medium, genes, and the like. The invention relates to a fine particle and a pharmaceutical preparation as so-called drug delivery system. More particularly, for example, the invention relates to a fine particle and a pharmaceutical preparation which effectively enclose protein, peptide drugs, and nucleic acid drugs of high hydrophilic property and large molecular weight.

### BACKGROUND ART

Particulate preparations having drugs enclosed in fine particles are developed, and are attempted to be used as drug carriers. These fine particles are called microparticle, microsphere, microcapsule, nanoparticle, or nanosphere.

In fine particles such as usual particles composed of biodegradable polymers and polymer micelles, the compartment for enclosing the drugs is in hydrophobic environment, and the efficiency of enclosing hydrophilic drugs in fine particles is low. On the other hand, fine particles formed of lipid such as liposome are poor in drug enclosing efficiency, and it is hard to control the release of the enclosed drugs. In particular, it is hard to enclose protein and peptide drugs of high hydrophilic property and large molecular weight in the fine particles while maintaining the bioactivity.

However, there is a problem that protein, peptide drugs, and nucleic acid drugs are hydrolyzed by decomposing enzymes existing in the body. It is demanded to avoid decomposition by enzymes and stabilize in the body by preparing fine particles by enclosing protein and peptide drugs inside of the fine particles. As Florence et al. demonstrated in the experiment by using fine particles not enclosing drugs, absorption into the intestinal tract is promoted when the particle size is smaller (see non-patent literature 1). By enclosing protein, peptide drugs, and nucleic acid drugs in micro-sized fine particles, it is expected to be applied in other method of administration than painful injection.

To solve this problem, as fine particles enclosing hydrophilic drugs such as protein and peptide drugs, it has been proposed to form fine particles after forming so-called reverse micelles in an organic solvent adding an amphiphilic polymer using aqueous solution of hydrophilic drugs (patent literature 1, 2).

In patent literature 1, an amphiphilic block copolymer having polyamino acid as hydrophilic segment and biodegradable polymer as hydrophobic segment is used to form a reverse phase micelle, and fine particles consisting of hydrophilic environment in the inner layer and hydrophobic environment in the outer layer are disclosed. However, the polyamino acid is known to frequently show a strong antigenicity. When protein is selected as the drug to be enclosed, many polyamino acids capable of forming hydrophilic segments have electric charges, and when a charged polymer is used, it is known that the bioactivity of protein is sacrificed. If polyamino acids are polymerized from amino acid N-carboxy anhydride monomer, polymerization must be operated in dehydrating condition.

Patent literature 2 relates to cosmetics and detergents containing particles having functional substances enclosed in a block polymer, in which a reverse phase micelle is formed by using amphiphilic block copolymer of vinyl ether system, and fine particles consisting of hydrophilic environment in the inner layer and hydrophobic environment in the outer layer are disclosed. In the fine particles, since the amphiphilic block copolymer of vinyl ether system is not biodegradable, it cannot be applied to injection, oral administration or other internal administration. Besides, since the outer layer is hydrophobic, it is hard to prepare these fine particles in water-based dispersant preferably administered internally.
Patent literature 1: Japanese Patent Application Laid-Open No. 11-269097
Patent literature 2: Japanese Patent Application Laid-Open No. 2004-18438
Non-patent literature 1: P.U. Jani et 3 al., "Nanoparticle uptake by the rat gastrointestinal mucosa: quantitation and particle size dependency" (Journal of Pharmacy and Pharmacology, 1990, vol. 42, No. 12, pp. 821-826).

### DISCLOSURE OF THE INVENTION

A first invention aspect of the present inventions is characterized by the following constitution.

A fine particle comprising an amphiphilic polymer, further comprising an inner nucleus of hydrophilic segment of amphiphilic polymer, and a hydrophobic outer layer of hydrophobic segment of amphiphilic polymer, and having a surface modifier bonded to the hydrophobic outer layer.

A second invention of the present inventions is characterized by the following constitution.

A fine particle comprising an inner nucleus of hydrophilic segment of amphiphilic polymer, and a hydrophobic outer layer of hydrophobic segment of amphiphilic polymer, in which:
(A) the hydrophobic segment of amphiphilic polymer comprising any one selected from the group consisting of aliphatic polyester, polyorthoester, polycyano-acrylic ester, polyalkylene glycol, polyurethane, polysiloxane, polyamino acid, and poly acid anhydride, and
(B) the hydrophilic segment of amphiphilic polymer comprising any one selected from the group consisting of polyethylene glycol, polyvinyl pyrrolidone, polyvinyl alcohol, polyethylene imine, polyacrylic acid, polymethacrylic acid, poly-1,3-dioxolane, 2-methacryloyl oxyethyl phosphoryl choline polymer, poly-1,3,6-trioxane, and polysaccharides.

### BRIEF DESCRIPTION OF THE DRAWINGS

Fig. 1 shows an example of particle size distribution of fine particles composed of polyethylene glycol-poly (epsilon-caprolactone) enclosed with horse radish peroxidase.
Fig. 2 shows changes of particle size of fine particles depending on the adding concentration of surface modifier.
Fig. 3 shows changes of particle size depending on the molecular weight of poly (epsilon-caprolactone) of amphiphilic block copolymer.
Fig. 4 shows the enclosing efficacy of medicinal insulin enclosed in fine particles in various conditions.
Fig. 5 shows sustained-release behavior of protein from fine particles in physiological conditions.
Fig. 6 shows changes of particle size of fine particles depending on the adding concentration of surface modifier.
Fig. 7 shows changes of glucose concentration in blood by hypodermic injection of insulin-enclosed fine particles in mouse.

### BEST MODE FOR CARRYING OUT THE INVENTION

A first invention of the present inventions is explained below.

The first invention of the present inventions relates to a fine particle comprising an amphiphilic polymer, further comprising an inner nucleus of hydrophilic segment of amphiphilic polymer, and a hydrophobic outer layer of hydrophobic segment of amphiphilic polymer, and having a surface modifier bonded to the hydrophobic outer layer.

The amphiphilic polymer of the present invention is comprised at least two segments, and at least one segment is hydrophilic and at least one segment is hydrophobic. A segment is said to be hydrophilic when solubility in water of this segment is higher than other segments. A hydrophilic segment is desirably soluble in water, but if less soluble, it is hydrophilic as far as its solubility in water is higher than other segments. A segment is said to be hydrophobic when solubility in water of this segment is lower than other segments. A hydrophobic segment is desirably insoluble in water, but if soluble, it is hydrophobic as far as its solubility in water is lower than other segments.

The hydrophilic segment of amphiphilic polymer of a fine particle of the invention is preferred to be biocompatible high polymer. In the invention, the biocompatible high polymer is a substance not having an extreme harm when administered in the body. More specifically, in the case of oral administration of high polymer in rat, the LD 50 is 2,000 mg/kg or more.

The hydrophilic segment of amphiphilic polymer of a fine particle of the invention, is not particularly limited.

The hydrophilic segment of amphiphilic polymer of a fine particle of the invention comprises any one selected from the group consisting of polyethylene glycol, polyvinyl pyrrolidone, polyvinyl alcohol, polyethylene imine, polyacrylic acid, polymethacrylic acid, poly-1,3-dioxolane, 2-methacryloyl oxyethyl phosphoryl choline polymer, poly-1,3,6-trioxane, polyamino acid, and polysaccharides.

More preferable hydrophilic segment of amphiphilic polymer of a fine particle of the invention is polyethylene glycol, polyvinyl pyrrolidone, polyvinyl alcohol, polyethylene imine, polyacrylic acid, polymethacrylic acid, poly-1,3-dioxolane, 2-methacryloyl oxyethyl phosphoryl choline polymer, poly-1,3,6-trioxane, polyamino acid, or polysaccharides.

The hydrophilic segment of amphiphilic polymer of a fine particle of the invention preferably comprises an analog of any one selected from the group consisting of polyethylene glycol, polyvinyl pyrrolidone, polyvinyl alcohol, polyethylene imine, polyacrylic acid, polymethacrylic acid, poly-1,3-dioxolane, 2-methacryloyl oxyethyl phosphoryl choline polymer, poly-1,3,6-trioxane, polyamino acid, and polysaccharides.

More preferably, the hydrophilic segment of amphiphilic polymer of a fine particle of the invention is an analog of any one selected from the group consisting of polyethylene glycol, polyvinyl pyrrolidone, polyvinyl alcohol, polyethylene imine, polyacrylic acid, polymethacrylic acid, poly-1,3-dioxolane, 2-methacryloyl oxyethyl phosphoryl choline polymer, poly-1,3,6-trioxane, polyamino acid, and polysaccharides.

The hydrophilic segment of amphiphilic polymer of a fine particle of the invention comprises a copolymer of any one selected from the group consisting of polyethylene glycol, polyvinyl pyrrolidone, polyvinyl alcohol, polyethylene imine, polyacrylic acid, polymethacrylic acid, poly-1,3-dioxolane, 2-methacryloyl oxyethyl phosphoryl choline polymer, poly-1,3,6-trioxane, polyamino acid, and polysaccharides.

More preferably, the hydrophilic segment of amphiphilic polymer of a fine particle of the invention is a copolymer of any one selected from the group consisting of polyethylene glycol, polyvinyl pyrrolidone, polyvinyl alcohol, polyethylene imine, polyacrylic acid, polymethacrylic acid, poly-1,3-dioxolane, 2-methacryloyl oxyethyl phosphoryl choline polymer, poly-1,3,6-trioxane, polyamino acid, and polysaccharides.

In the hydrophilic segment of amphiphilic polymer of a fine particle of the invention, polyamino acid include, for example, polyaspartic acid, polyglutamic acids, poly lysine, polyarginine, polyserine, polythreonine, polytyrosine, polyhistidine, polycysteine, polyasparagine, and polyglutamine. Polysaccharides include cellulose, chitin, chitosan, gellan gum, arginic acid, hyaluronic acid, pullulan, and dextran.

The molecular weight of hydrophilic segment of amphiphilic polymer is preferred to be 50,000 or less, and more preferably 5,000 or less.

The hydrophobic segment of amphiphilic polymer of a fine particle of the invention is not particularly limited.

The hydrophobic segment of amphiphilic polymer of a fine particle of the invention preferably comprises any one selected from the group consisting of polyester, polyorthoester, polycyano-acrylic ester, polyether, polyvinyl, polyurethane, polysiloxane, polyamino acid, and poly anhydride.

The hydrophobic segment of amphiphilic polymer of a fine particle of the invention is preferably any one selected from the group consisting of polyester, polyorthoester, polycyano-acrylic ester, polyether, polyvinyl, polyurethane, polysiloxane, polyamino acid, and poly anhydride.

The hydrophobic segment of amphiphilic polymer of a fine particle of the invention preferably comprises an analog of any one selected from the group consisting of aliphatic polyester, polyorthoester, polycyano-acrylic ester, polyalkylene glycol, polyurethane, polysiloxane, polyamino acid, and poly acid anhydride.

The hydrophobic segment of amphiphilic polymer of a fine particle of the invention is preferably an analog of any one selected from the group consisting of aliphatic polyester, polyorthoester, polycyano-acrylic ester, polyalkylene glycol, polyurethane, polysiloxane, polyamino acid, and poly acid anhydride.

In the hydrophobic segment of amphiphilic polymer of a fine particle of the invention, polyester is preferably aliphatic polyester from the viewpoint of biodegradable properly as discussed below, and preferred examples of polyether include polypropylene glycol and polyalkylene glycol.

Specific examples of hydrophobic segment of amphiphilic polymer of the fine particle of the invention include polyglycolic acid, polylactic acid, poly (2-hydroxy butyric acid), poly (2-hydroxy valeric acid), poly (2-hydroxy capronic acid), poly (2-hydroxy capric acid), poly (malic acid), poly (citric acid), polybenzyl malolacnate, polymalite benzylester, poly {3-[(benzyloxy carbonyl) methyl[-1,4-dioxane-2,5-dion]], poly (β-propiolactone), poly (δ-valerololactone), poly (ε-caprolactone), poly (N-benzyl oxycarbonyl-L-serine-beta-lactone), poly [1,3-bis (p-carboxy phenoxy) methane], poly (terephthalic acid-sebacic acid anhydride), poly {3,9-bis (ethylidene-2,4,8,10-tetraoxa spiro [5,5]undecane-1,6-hexane diol), poly-a-cyanoacryilc acid isobutyl, polypropylene oxide, polyalkyl methacrylate, polyvinyl acetate, polysiloxane, polyalanine, polyleucine, polyisoleucine, polyvaline, polyproline, polyphenyl alanine, polymethionine, and their high polymer compound derivatives, and copolymers.

In the hydrophobic segment of amphiphilic polymer of a fine particle of the invention is preferably a biocompatible high polymer. In the invention, the biocompatible high polymer is a substance not having an extreme harm when administered in the body, and more specifically, in the case of oral administration of high polymer in rat, the LD 50 is 2,000 mg/kg or more.

In the hydrophobic segment of amphiphilic polymer of a fine particle of the invention is preferably not to have an extreme harm when administered in the body, and is more preferably a biodegradable high polymer. In the invention, the biodegradable high polymer is the high polymer that is metabolized and lowered in molecular weight after being administered in the body, and then excreted from the body.

The hydrophobic segment of amphiphilic polymer of a fine particle of the invention is preferred to comprise any one selected from the group consisting of poly (epsilon-caprolactone), polylactic acid, and polyglycolic acid.

The hydrophobic segment of amphiphilic polymer of a fine particle of the invention is preferably any one selected from the group consisting of poly (epsilon-caprolactone), polylactic acid, and polyglycolic acid.

The hydrophobic segment of amphiphilic polymer of a fine particle of the invention is also preferably a copolymer such as poly (lactic acid-glycolic acid), or poly (lactic acid-epsilon-caprolactone).

The molecular weight of the hydrophobic segment of amphiphilic polymer of a fine particle of the invention is preferred to be in a range of 1000 to 100,000.

The structure of the amphiphilic polymer of a fine particle of the invention is not particularly limited, but is preferred to be a copolymer having a structure of any one selected from the group consisting of block structure, graft structure, and branch structure. In the case of block structure, the sequence of hydrophilic segment/hydrophobic segment (A/B) is preferably AB structure, ABA structure, or BAB structure. In the case of block copolymer of three or more components, plural hydrophilic segments and hydrophobic segments may coexist. In the case of graft structure, either one of hydrophilic segment and hydrophobic segment is the principal chain, and the other is preferred to be graft chain.

In the amphiphilic polymer of the invention, the ratio of average molecular weight of hydrophobic segment to average molecular weight of hydrophilic polymer is preferably 0.1 times to 10 times, or more preferably 2 times to 7 times.

The mean particle size of fine particle in the invention is preferred to be 25 to 5000 nanometers, more preferably 50 to 1000 nanometers, or most preferably 150 nanometers or less. The mean particle size of fine particle can be measured directly by using a laser scatter type particle size distribution meter (for example, Microtrac ASVR/Mictrotrac HRA (9320-X100)).

The shape of fine particle in the invention is not particularly limited, but may include spherical, elliptical, and amorphous shape.

The present invention is a fine particle which comprises amphiphilic polymer having a surface modifier bonded to the hydrophobic outer layer of the fine particle.

The surface modifier of the invention is bonded to the hydrophobic outer layer of a fine particle comprising amphiphilic polymer, and the bond may be either non-covalent bond or covalent bond. Non-covalent bond is preferably hydrophobic interaction, or may be electrostatic interaction, hydrogen bond, van der Waals force, or combined bond of them. In non-covalent bond, preferably, the hydrophobic outer layer of a fine particle comprising amphiphilic polymer, and the hydrophobic portion of the surface modifier mentioned below is bonded by hydrophobic interaction. In this case, more preferably, the dispersion medium of a fine particle is water, buffer solution, physiological saline, aqueous solution containing surface modifier or hydrophilic solvent.

The surface modifier of the invention is preferably bonded to the hydrophobic outer layer of a fine particle comprising amphiphilic polymer by non-covalent bond.

The surface modifier of the invention is preferably bonded to the hydrophilic outer layer of a fine particle comprising amphiphilic polymer by covalent bond. Covalent bond is preferably bonding to terminal end or side chain of hydrophobic segment of amphiphilic polymer. Covalent bond is not particularly limited, but may include amide bond, ester bond, urethane bond, disulfide bond, sulfide bond, and hydrazone bond.

In the invention, the surface modifier may be bonded to the surface after forming a fine particle, or a fine particle may be formed after the surface modifier is bonded to amphiphilic polymer.

The surface modifier of the invention is preferably present at a position capable of contacting with a substance existing in the solvent or outside of a fine particle.

The surface modifier of the invention is a compound preferably having a property of enhancing the colloid stability of a fine particle, or a property of giving effects on the kinetics of a fine particle after administration in the body, or both properties. The surface modifier may be either a single material or a mixture of plural types. Herein, the property of enhancing the colloid stability is to prevent or retard the aggregation of fine particles in the solvent. The compound capable of giving effects on the kinetics of fine particles after administration in the body is not particularly limited, and includes a substance sticking to the surface of biological barrier, invading into the barrier, or promoting crossing of the barrier. In particular, a compound promoting crossing of the mucosa or epithelial cell layer existing in the digestive tract is preferred. The compound capable of giving effects on the kinetics of fine particles in the body may be any compound capable of decreasing interaction of fine particles with substances existing in the body, such as protein and cells.

The surface modifier of the invention is not particularly limited, and is preferred to be hydrophilic polymer.

The surface modifier of a fine particle in the invention is preferably any hydrophilic polymer selected from the group consisting of polyethylene glycol, polyvinyl pyrrolidone, polyvinyl alcohol, polyethylene imine, polyacrylic acid, polymethacrylic acid, poly-1,3-dioxolane, 2-methacryloyl oxyethyl phosphoryl choline polymer, poly-1,3,6-trioxane, polyamino acid, protein, and polysaccharides.

The hydrophilic polymer of surface modifier of a fine particle in the invention is preferably an analog of any one selected from the group consisting of polyethylene glycol, polyvinyl pyrrolidone, polyvinyl alcohol, polyethylene imine, polyacrylic acid, polymethacrylic acid, poly-1,3-dioxolane, 2-methacryloyl oxyethyl phosphoryl choline polymer, poly-1,3,6-trioxane, polyamino acid, protein, and polysaccharides.

The analog of hydrophilic polymer includes a surfactant of hydrophilic polymer partly modified with long-chain alkyl and hydrophobic group, but is not particularly limited.

The surface modifier of a fine particle in the invention is preferred to be equivalent to Pluronic manufactured and distributed by BASF (a registered trademark of BASF).

The polyamino acid of surface modifier of fine particle in the invention is preferably polyaspartic acid, polyglutamic acid, or analog thereof. A particularly preferred example is an analog prepared by introducing long-chain alkyl group in part of polyaspartic acid or polyglutamic acid.

The protein of the surface modifier of a fine particle of the invention is preferred to be gelatin, casein, or albumin for purpose of enhancing the dispersion property of particles.

The polysaccharide of the surface modifier of a fine particle of the invention is preferably cellulose, chitin, chitosan, gellan gum, arginic acid, hyaluronic acid, pullulan, or dextran, and in particular cholesterol-bearing pullulan is desired for enhancing the dispersion property of particles.

The polysaccharide of the surface modifier of a fine particle of the invention is preferably an analog of any one selected from the group consisting of cellulose, chitin, chitosan, gellan gum, arginic acid, hyaluronic acid, pullulan, and dextran.

In the invention, the surface modifier is preferably an amphiphilic compound.

In the surface modifier of a fine particle of the invention, the amphiphilic compound is preferably a lipid.

In the surface modifier of a fine particle of the invention, the amphiphilic compound is preferably a surfactant.

In the surface modifier of a fine particle of the invention, the surfactant is preferably polyoxylene-polypropyleneglycol copolymer, sucrose fatty acid ester, polyethylene glycol fatty acid ester, polyethylene sorbitan mono-fatty acid ester, polyoxyethylene sorbitan di-fatty acid ester, polyoxy ethylene glycerin mono-fatty acid ester, polyoxy ethylene glycerin di-fatty acid ester, polyglycerin fatty acid ester, polyoxy ethylene castor oil, polyoxy ethylene cured castor oil, other nonionic active agent, lauryl sodium sulfate, lauryl ammonium sulfate, stearyl sodium sulfate, other alkyl sulfate or lecithin.

The surface modifier of a fine particle of the invention is preferably peptide, protein, sugar, or its analog, and, for example, targeting antibody or basic peptide is preferred. The peptide, protein or sugar is particularly preferred to be an analog of long-chain alkyl or other hydrophobic group modified in part, or an analog modified hydrophilic polymer or amphiphilic compound.

The bonding amount of surface modifier of fine particle of the invention is preferably 0.0001 to 1 % of the particle weight.

A second invention of the present inventions is explained.

The second invention of the inventions relates to a fine particle comprising an inner nucleus of hydrophilic segment of amphiphilic polymer, and a hydrophobic outer layer of hydrophobic segment of amphiphilic polymer, wherein:
(A) the hydrophobic segment of amphiphilic polymer comprising any one selected from the group consisting of aliphatic polyester, polyorthoester, polycyano-acrylic ester, polyalkylene glycol, polyurethane, polysiloxane, polyamino acid, and polyacid anhydride, and
(B) the hydrophilic segment of amphiphilic polymer comprising any one selected from the group consisting of polyethylene glycol, polyvinyl pyrrolidone, polyvinyl alcohol, polyethylene imine, polyacrylic acid, polymethacrylic acid, poly-1,3-dioxolane, 2-methacryloyl oxyethyl phosphoryl choline polymer, poly-1,3,6-trioxane, and polysaccharides.

The amphiphilic polymer is particularly preferred to be composed of biocompatible or biodegradable segment. Examples of combination of hydrophilic segment/hydrophobic segment of such amphiphilic polymer preferably include polyethylene glycol/poly (epsilon-caprolactone), polyethylene glycol/poly (lactic acid) copolymer, polyvinyl pyrrolidone/poly (lactic acid), and dextran/poly (lactic acid).

In the second invention of the present inventions, the structure of amphiphilic polymer is not particularly limited, but is preferred to be a copolymer having a structure of any one of block structure graft structure, and branch structure. In the case of block structure, the sequence of hydrophilic segment/hydrophobic segment (A/B) is preferably AB structure, ABA structure, or BAB structure. In the case of graft structure, either one of hydrophilic segment and hydrophobic segment is the principal chain, and the other is preferred to be graft chain.

In the second invention of the inventions, the ratio of average molecular weight of hydrophobic segment to average molecular weight of hydrophilic polymer is preferably 0.1 times to 10 times, or more preferably 2 times to 7 times.

In the second invention of the inventions, the mean particle size of fine particle is preferred to be 25 nm to 5 µm, more preferably 50 nm to 1000 nm.

A fine particle of the inventions may contain a drug, whether in the first invention or in the second invention. The drug may be either one kind of a mixture of two or more kinds.

A fine particle of the inventions contains, preferably, a drug in its inner nucleus composed of hydrophilic segment of amphiphilic polymer.

A fine particle of the inventions is preferably used as medicinal compositions containing fine particles including hydrophilic drugs. The hydrophilic drugs in the invention are those distributed in the hydrophilic inner nucleus of particles. The hydrophilic drugs are not particularly limited, but may include examples such as low molecular compound, protein, peptide, DNA, RNA, modified nucleic acid, and contrast medium for diagnostic purpose. In the medicinal composition containing the fine particle of the invention, the hydrophilic drug is preferred to be a medicinal composition containing a fine particle selected from any one the group consisting of protein, peptide, DNA, RNA, and modified nucleic acid. If the drug is a hydrophobic drug, by making it soluble in water by using a solubilizing agent, it may be contained in a fine particle. As a solubilizing agent, cyclodextrin or its analog is particularly preferred.

The bioactive protein used in the medicinal composition containing a fine particle of the inventions includes peptide hormone, bioactive protein, enzyme protein, and antibody. Examples include parathyroid hormone (PTH), calcitonin, insulin, angiotensin, glucagon, gastrin, growth hormone, prolactin (luteotropic hormone), gonadotropin (gonandotropic hormone), cyclotropic hormone, adrenocorticotropic hormone, melanin cell stimulating hormone, vasopressin, oxytocin, prothylerin, leuteinizing hormone (LH), corticotropin, somatropin, tyrotropin (thyroid stimulating hormone), stomatostatin (growth hormone stimulating factor), hypothalamic hormone (GnRH), G-CSF, erythropoetin, HGF, EGF, VEGF, interferon α, interferon β, interferon γ, interleukins, FGF (fibroblast growth factor), BMP (bone marrow protein), superoxide dismutase (SOD), urokinase, lisozyme, and vaccines. These bioactive proteins may be either natural proteins or peptides, or derivatives modified partly in sequence, or modified with polyethylene glycol or sugar chain. The nucleic acid may be complexed with cationic surfactant, cationic lipid, cationic polymer, or analogs thereof.

A fine particle of the inventions may contain amphiphilic polymer, and substances other than drugs.

Substances to be contained in a fine particle of the inventions are not particularly limited, and substances having effects on particle size, particle structure, particle stability, stability of amphiphilic polymer, stability of drugs, or bioactivity of drugs may be added.

A fine particle of the inventions may contain additives applied in either particle inner nucleus or particle outer layer. Additive usable in the fine particle of the inventions are not particularly limited, and is buffer, antioxidant, salt, polymer or sugar.

The medicinal composition containing a fine particle of the inventions may contain a fine particle containing buffer, antioxidant, salt, polymer or sugar.

Additives usable in the fine particle of the inventions are, for example, water, organic solvents permitted pharmaceutically, collagen, polyvinyl alcohol, polyvinyl pyrrolidone, carboxyvinyl polymer, carboxy methyl cellulose sodium, sodium polyacrylate, sodium arginate, water soluble dextran, carboxy methyl starch sodium, pectin, methyl cellulose, ethyl cellulose, xanthan gum, gum arabic, casein, gelatin, agar, diglycerin, propylene glycol, polyethylene glycol, vaseline, paraffin, stearyl alcohol, stearic acid, human serum albumin (HSA), mannitol, sorbitol, lactose, and surfactant permitted as medical additive.

The pharmaceutical preparation containing a fine particle of the inventions is available in various dosage forms, such as powder form consisting of pharmaceutical preparation containing fine particle of the invention and pharmacologically allowed additive, mixture of particulate preparation with water or other medium, liquid form of mixture with polysaccharide, water or other medium and pharmacologically allowed base, or solidified or semi-solidified form by combination of particulate preparation and pharmacologically allowed base. The solidified dosage form is preferably capsule (hard capsule, soft capsule, etc.), granules or dust, and the semi-solidified dosage form is preferably a form of fine particles dispersed in hydro gel, or a form dispersed in vaseline, polyethylene glycol, or the like. PVA gel, N-isopropyl acylamide gel, gelatin gel, agar gel, or cellulose hydro gel is especially preferred as hydro gel.

Examples of the base include various organic and inorganic substances generally used in pharmaceutical materials, such as vehicle, lubricant, binder, disintegrator, solvent, solvent aid, emulsifier, isotonic agent, buffer, analgesic, and absorption promoter.

Dispersion medium of a fine particle of the inventions is preferably water, aqueous solution, water miscible liquid, and preferred examples are glycerin, polyethylene glycol, and pharmacologically allowed nonaqueous solution. The aqueous solution may be either non-isotonic or isotonic solution. The aqueous solution may be prepared by mixing water and one or plural types of components, for example, glycerin, mannose, glucose, fructose, xylose, trehalose, mannit, sorbit, xylit, or polyethylene glycol, or other polyol or sodium chloride or electrolyte. The nonaqueous solvent includes acetyl glycerin fatty acid ester, liquid sugar, oleic acid, orange oil, reduced malt sugar syrup, triethyl citrate, high fructose liquid sugar, high glucose syrup, wheat germ sugar, safflower oil, safflower oil fatty acid, snake oil, dimethyl polysiloxane, ginger oil, soy lecithin, medium chain fatty acid triglyceride, natural vitamin B, triacetin, trioleic acid sorbitan, piperonyl butoxide, sun flower oil, phytic acid, diethyl phthalate, dibutyl phthalate, butyl phthalyl butyl glycolate, glucose-fructose sugar, polysorbate, starch syrup, myristic acid octyl dodecyl, cotton seed oil, monooleic acid sorbitan, ethyl butyrate, phosphoric acid, and lemon oil.

A fine particle of the invention is manufactured in various processes, such as (a) a process of dissolving amphiphilic polymer in organic solvent, and stirring and adding drug aqueous solution in the polymer solution, (b) a process of displacing the organic solvent with other solvent, and (c) a process of bonding the surface modifier to the surface of fine particles, but the manufacturing method is not limited to these processes alone.

Hydrophobic segment of the amphiphilic polymer is preferred to be soluble in the organic solvent and hydrophilic segment hardly soluble or insoluble, but both segments may be hardly soluble or both segments may be soluble. The method of bonding the surface modifier to the outer layer of fine particle consisting of the inner nucleus of hydrophilic segment of amphiphilic polymer, and hydrophobic outer layer of hydrophobic segment is realized by, for example, adding the surface modifier after forming fine particles, and bonding to the outer layer of fine particles, or by once isolating the fine particles by freeze-drying, and adding the isolated fine particles to the dispersion medium containing surfactant.

When the fine particle of the inventions is used as medicine, the organic solvent is preferably made of a material not having extreme effects on the body. The organic solvent is not particularly limited, but preferred examples include acetonitrile, chlorobenzene, chloroform, methylene chloride, carbon tetrachloride, cyclohexane, 1,2-dichloroethene, dichloromethane, 1,2-dimethoxy ethane, N,N-dimethyl acetamide, N,N-dimethyl formamide, 1,4-dioxane, 2-ethoxy ethanol, ethylene glycol, formamide, hexane, methanol, 2-methoxy ethanol, methyl butyl ketone, methyl cyclohexane, N-methyl pyrrolidone, nitromehane, pyridine, sulfolane, tetralin, toluene, 1,1,2-trichloroethene, xylene, acetic acid acetone, annisol, 1-butonol, 2-butanol, acetic acid n-butyl, t-butyl methyl ether, cumene, dimethyl sulfoxide, ethanol, ethyl acetate, diethyl ether, ethyl formate, formic acid, heptane, isobyutyl acetate, isopropyl acetate, methyl acetate, dimethyl carbonate, 3-methyl-1-butanol, methyl ethyl ketone, methyl isobutyl ketone, 2-methyl-l-propanol, pentane, 1-pentanol, 1-propanol, 2-propanol, propyl acetate, tetrahydrofurane, and mixed solvent thereof.

The method of dissolving the amphiphilic polymer in organic solvent is realized by, for example, stirring, heating, ultrasonic processing, or dialysis, but is not limited to these examples.

The particle size of a fine particle of the inventions can be controlled by structure of amphiphilic polymer, concentration of amphiphilic polymer, content of medicinal aqueous solution, composition of organic solvent, stirring speed, or additives to medicinal aqueous solution. Additives may be directly added to the medicinal aqueous solution, or may be separately added as additive aqueous solution, or added to organic solvent.

After forming fine particles, the inner nucleus water can be eliminated by evaporating the inner nucleus water, or displacing the organic solvent for particle forming with a solvent compatible with water. The formed fine particles are collected by freeze-drying, filtering, centrifugal operation, or re-sedimentation.

If the organic solvent for particle forming is not suited to freeze-drying process, it may be displaced with a solvent suited to freeze-drying, and the solvent displacing process may be realized in other processing of adding solvent than the solvent removing process. The particle dispersion solution may be merely added to other solvent. Solvents suitable to freeze-drying include dioxane, acetic acid, tert-butanol, dimethyl carbonate, morpholine, paraxylene, and cyclohexane. The solvent removing method includes, for example, evaporation, dialysis, freeze-drying, centrifugal operation, filtering, and re-sedimentation.

The surface modifier can be presented on the surface of fine particles by, for example, chemical bonding to the terminal end of hydrophobic segment of amphiphilic polymer. For example, after forming fine particles from amphiphilic polymer, the surface modifier can be bonded to the surface of fine particles. The method of bonding the surface modifier after preparation of fine particles is not particularly limited, and includes a method of adding fine particles to the solvent containing the surface modifier.

A fine particle of the invention is preferred to be fine particle containing water as one of the components of the inner nucleus, and the fine particle is preferably prepared by evaporating the water in the fine particle.

The administration method and application field of the pharmaceutical preparation containing the fine particle of the inventions are not particularly limited, but are widely applicable. For example, the pharmaceutical preparation can be administered in various routes, including oral administration, parenteral administration, enteric administration, pulmonary administration, local administration (nose, skin, eye), and body cavity administration.

Parenteral administration includes the following routes.
(1) Intravenous administration [to target on the liver, spleen, or bone marrow by circulating the fine particles for controlling and releasing the action substance, for example, peptide drug, cell growth suppressant, immune stimulant, growth factor, for example, colony stimulating factor (leukocyte regulating factor), growth factor, etc., in blood].
(2) Intramuscular administration [to administer depot preparation for applying action substance, for example, peptide drug or hormone for a long period].
(3) Intraarticular administration [to administer antirheumatoid or immune suppressor, for example, for remedy of arthritis].
(4) Intravacuolar administration [to administer cell growth suppressor or peptide drug, for example, for remedy of carcinoma in peritoneum or pleural cavity].]
(5) Subcutaneous administration [to administer depot preparation of cell growth inhibitor, for example, for remedy of skin cancer].

For enteric administration, in particular, the following aspects should be taken into consideration.
(1) Administration of vitamins.
(2) Adsorption of lymphatic fluid (for example, targeting on lymph node by action substance such as cell growth inhibitor).
(3) Administration of antigen (for example, oral immunization by making use of Peyer's patch).
(4) Uptake of peptide drug by making use of M cell.

For pulmonary administration, in particular, the following aspects should be taken into consideration.
(1) Aerosol preparation or dispensed aerosol preparation (spraying of aqueous dispersion solution of particulate preparation).
(2) Drip infusion of the dispersion solution.

For local administration, for example, the following aspects should be taken into consideration.
- Administration of skin remedy, such as corticoid or antimycotic.
- Administration of periodontal remedy in periodontal pocket.
- Eye drops or ocular gel of beta blocker.
- Cosmetic analogous to liposome preparation.

The invention realizes improvement of stability and behavior of protein in the body. The invention enables oral and enteric administration of bioactive protein, and as compared with the injection administration method, simple and less painful medication for patient is realized, and the conventional concept of protein preparation is drastically changed, which is expected to development of completely new preparations.

For hypodermic injection of protein, by using the fine particles of the invention, long-term release is possible, and as compared with the conventional administration method, it is excellent economically, and patient's burden is lessened dramatically.

Examples are shown below, but the invention is not limited to these examples alone.

### Example 1

### Manufacture of fine particles

10 mg of polyethylene glycol-poly (epsilon-caprolactone), which is an amphiphilic polymer, (molecular weight of polyethylene glycol is 5,000 and molecular weight of poly (epsilon-caprolactone) is 10,000) was dissolved in 500 µL of chloroform. This solution was added to 2 mL of hexane, chloroform mixed solution (hexane 1.6 mL, 0.4 mL), and a polymer solution was prepared. While the polymer solution was stirred at stirring speed of 1,600 rpm, and 10-100 µL of bovine serum albumin solution was dropped. The solution was stirred for 5 minutes, and fine particles were manufactured. The particulate dispersion solution was developed on a silicon wafer, and dried sufficiently in vacuum, and platinum was evaporated, and the particles were observed by scanning electron microscope (HITACHI S-4800).

### <Results>

Fine particles of several micrometers to hundred micrometers in diameter were observed. The shape of particles observed after drying was not contradictory to the structure of fine particles having protein existing in particles covered with polymer outer layer.

### Example 2

### Manufacture of fine particles

10 mg of polyethylene glycol-poly (epsilon-caprolactone), which is an amphiphilic polymer, (molecular weight of polyethylene glycol is 5,000 and molecular weight of poly (epsilon-caprolactone) is 10,000or 37,000) was heated and dissolved in 100 µL of ethyl acetate. This polymer solution was stirred at stirring speed of 1,600 rpm, and 50-200 µL of horse radish peroxidase aqueous solution (1 wt.% horse radish peroxidase, 9 wt.% sucrose, 10 mM Tris-HCl pH 7.4) was dropped. The solution was further stirred for 1 hour, and fine particles were manufactured. The particle size of fine particles was measured by dynamic light scatter method by using apparatus Zetasizer 3000HSA (manufactured by Malvern Instruments). The obtained data was analyzed by CONTIN method and histogram method, and the particle size was calculated.

### <Results>

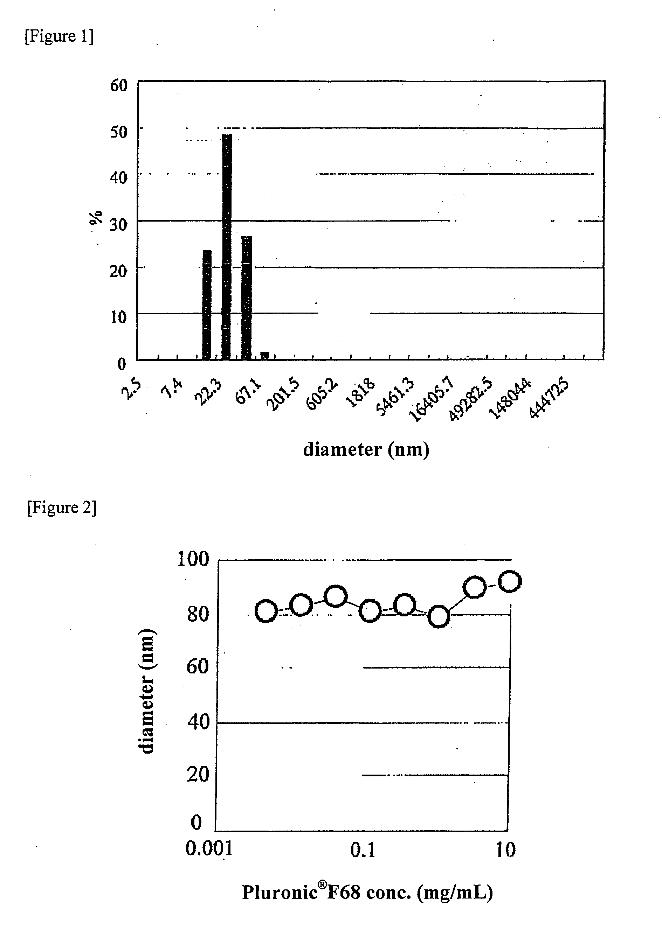

Fine particles of minimum number-average particle size of 25.1 nm were formed (Fig. 1). z-Average particle size was studied, and in polymer concentration range, it was changed from 200 nm to several microns.

### Example 3

### Manufacture of fine particles

10 mg of polyethylene glycol-poly (epsilon-caprolactone), which is an amphiphilic polymer, (molecular weight of polyethylene glycol is 5,000 and molecular weight of poly (epsilon-caprolactone) is 37,000) was dissolved in 2 mL of ethyl acetate, and a polymer solution was prepared. This polymer solution was stirred at stirring speed of 1,600 rpm, and 100 µL of 1 wt.% horse radish peroxidase aqueous solution was dropped. The solution was further stirred for 1 hour, and was added to 10 times in volume of dioxane. The solvent was evaporated, and the solution was concentrated to about 2 mL, and the particulate dispersion solution was added to phosphoric acid buffer solution (10 mL) adding Pluronic (registered trademark of BASF) F68 (PEO-PPO-PEO block polymer) at various concentrations. The particle size of fine particles was measured by dynamic light scatter method by using apparatus Zetasizer 3000HSA (manufactured by Malvern Instruments). The obtained data was analyzed by CONTIN method and histogram method, and the particle size was calculated.

### <Results>

By adding Pluronic (registered trademark of BASF) F68 (PEO-PPO-PEO block polymer) as surface modifier to the phosphoric acid buffer solution, stable fine particles were formed in water having adding Pluronic (registered trademark of BASF) F68 bonded to particle surface. From the results of measurement of light scatter, it was found that the particle size varies depending on the concentration of adding Pluronic (registered trademark of BASF) F68 (Fig. 2). At 1 mg/mL of adding Pluronic (registered trademark of BASF) F68, the particle size was about 95 nm, and it decreased along with concentration of adding Pluronic (registered trademark of BASF) F68, becoming about 80 nm at 0.002 mg/mL.

### Example 4

### Manufacture of fine particles

10 mg of polyethylene glycol-poly (epsilon-caprolactone), which is an amphiphilic polymer, (molecular weight of polyethylene glycol is 5,000 and molecular weight of poly (epsilon-caprolactone) is 37,000) was dissolved in 2 mL of ethyl acetate, and a polymer solution was prepared. This polymer solution was stirred at stirring speed of 1,600 rpm, and 100 µL of 1 wt.% insulin solution was dropped. The solution was further stirred for 1 hour, and was added to 10 times in volume of dioxane. The solvent was evaporated, and the solution was concentrated to about 2 mL, and the particulate dispersion solution was added to phosphoric acid buffer solution (10 mL) adding 1 wt.% polyvinyl alcohol. The solution was freeze-dried, and 2 mL of water was added, and the particles were dispersed again. The particle size of fine particles was measured by dynamic light scatter method by using apparatus Zetasizer 3000HSA (manufactured by Malvern Instruments). The obtained data was analyzed by CONTIN method and histogram method, and the particle size was calculated.

### <Results>

By using polyvinyl alcohol as surface modifier, fine particles enclosed with insulin as drum were prepared. The average particle size of the fine particles dispersed again after freeze-drying was 121 nm.

### Example 5

### Manufacture of fine particles

10 mg of polyethylene glycol-poly (epsilon-caprolactone), which is an amphiphilic polymer, (molecular weight of polyethylene glycol is 5,000 and molecular weight of poly (epsilon-caprolactone) is 5,000, 10,000, 16,300, 24,500, or 37,000) was dissolved in 2 mL of ethyl acetate, and a polymer solution was prepared. This polymer solution was stirred at stirring speed of 1,600 rpm, and 100 µL of 1 wt.% insulin solution was dropped. The solution was further stirred for 1 hour, and was added to 10 times in volume of dioxane. The solvent was evaporated, and the solution was concentrated to about 2 mL, and the particulate dispersion solution was added to phosphoric acid buffer solution (10 mL) adding 1 wt.% polyvinyl alcohol. The solution was freeze-dried, and 2 mL of water was added, and the particles were dispersed again. The particle size of fine particles was measured by dynamic light scatter method by using apparatus Zetasizer 3000HSA (manufactured by Malvern Instruments). The obtained data was analyzed by CONTIN method and histogram method, and the particle size was calculated.

### <Results>

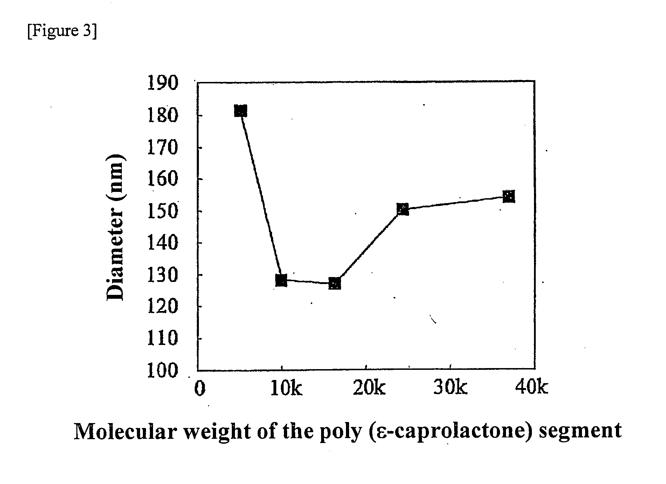

The particle size varied between 130 and 180 nm along with molecular weight of poly (epsilon-caprolactone) segment of amphiphilic block copolymer (Fig. 3).

### Example 6

### Manufacture of fine particles

10 mg of polyethylene glycol-poly (lactic acid)-polyethylene glycol, which is an amphiphilic polymer, (molecular weight of polyethylene glycol is 23,000 and molecular weight of poly (lactic acid) is 20,000) was dissolved in 2 mL of ethyl acetate, and a polymer solution was prepared. This polymer solution was stirred at stirring speed of 1,600 rpm, and 100 µL of 1 wt.% horse radish peroxidase solution was dropped. The solution was further stirred for 1 hour, and was added to 10 times in volume of dioxane. The solvent was evaporated, and the solution was concentrated to about 2 mL, and the particulate dispersion solution was added to phosphoric acid buffer solution (10 mL) adding 1 wt.% polyvinyl alcohol. The solution was freeze-dried, and 2 mL of water was added, and the particles were dispersed again. The particle size of fine particles was measured by dynamic light scatter method by using apparatus Zetasizer 3000HSA (manufactured by Malvern Instruments). The obtained data was analyzed by CONTIN method and histogram method, and the particle size was calculated.

### <Results>

By using polyethylene glycol-poly (lactic acid)-polyethylene glycol as amphiphilic polymer, fine particles enclosed with horse radish peroxidase were prepared. The average particle size of the fine particles dispersed again after freeze-drying was 75 nm.

### Example 7

### Determination of medicine content

10 mg of polyethylene glycol-poly (epsilon-caprolactone), which is an amphiphilic polymer, (molecular weight of polyethylene glycol is 5,000 and molecular weight of poly (epsilon-caprolactone) is 37,000) was dissolved in 2 mL of ethyl acetate, and a polymer solution was prepared. This polymer solution was stirred at stirring speed of 1,600 rpm, and 100 µL of 1 wt.% insulin solution (either containing or not containing 9 wt.% sucrose) was dropped. The solution was further stirred for 1 hour, and was added to 10 times in volume of dioxane. The solvent was evaporated, and the solution was concentrated to about 2 mL, and the particulate dispersion solution was added to phosphoric acid buffer solution (10 mL) adding 1 wt.% surface modifier Pluronic (registered trademark of BASF) F68 or polyvinyl alcohol. The solution was freeze-dried, and 2 mL of water was added, and the particles were dispersed again. The medicine enclosing rate was determined by quantitating the protein concentration after liquid chromatography measurement and ultrafiltration by Coumacy brilliant blue staining.

### <Results>

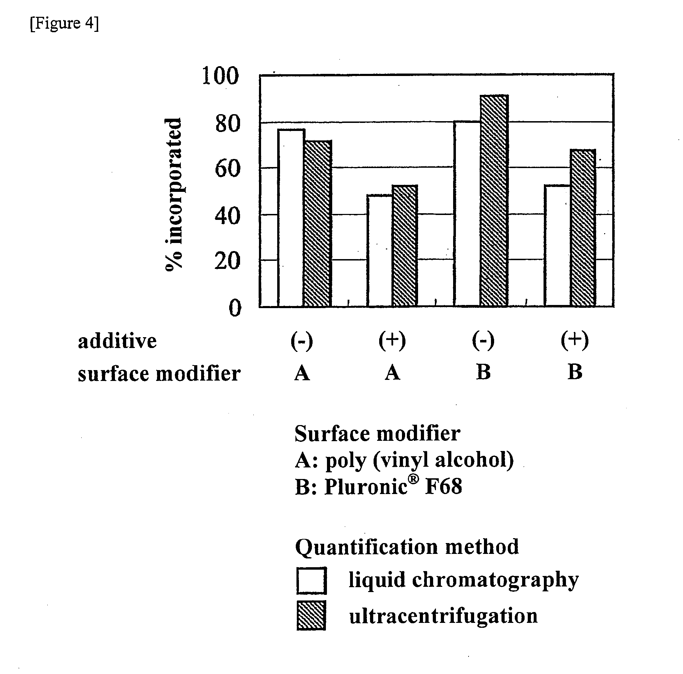

The contained amount of medicinal insulin was not changed by the type of surface modifier, but was changed depending on presence or absence of additive in the particles (Fig. 4). When using either modifier Pluronic (registered trademark of BASF) F68 or polyvinyl alcohol, the content was about 50% when sucrose was added, and about 80% when not added.

### Example 8

### Sustained release of medicine from fine particles

10 mg of polyethylene glycol-poly (epsilon-caprolactone), which is an amphiphilic polymer, (molecular weight of polyethylene glycol is 5,000 and molecular weight of poly (epsilon-caprolactone) is 37,000) was dissolved in 2 mL of ethyl acetate, and a polymer solution was prepared. This polymer solution was stirred at stirring speed of 1,600 rpm, and 100 µL of 1 wt.% serum albumin aqueous solution labeled with fluoresceine was dropped. The solution was further stirred for 1 hour, and was added to 10 times in volume of dioxane. The solvent was evaporated, and the solution was concentrated to about 2 mL, and the particulate dispersion solution was added to phosphoric acid buffer solution (10 mL) containing 1 wt.% surface modifier polyvinyl alcohol. The sample was freeze-dried, and 2 mL of water was added, and the particles were dispersed again. The sample was diluted 10 times in phosphoric acid buffer solution (pH 7.4), and incubated at 37 deg. C. By sampling in specified time, the amount of released protein was determined.

### <Results>

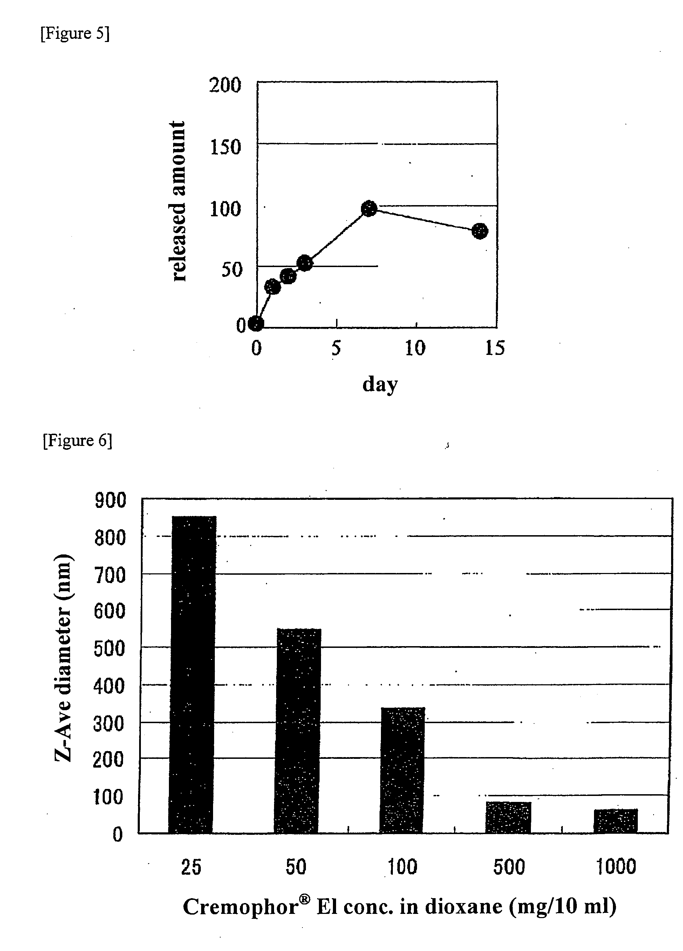

The fine particles were gradually decomposed in physiological condition, and the contained protein was released slowly (Fig. 5). The time scale of sustained release was in the unit of several days to several weeks.

### Example 9

### Manufacture of fine particles

10 mg of polyethylene glycol-poly (epsilon-caprolactone), which is an amphiphilic polymer, (molecular weight of polyethylene glycol is 5,000 and molecular weight of poly (epsilon-caprolactone) is 10,000) was dissolved in 2 mL of ethyl acetate, and a polymer solution was prepared. In this polymer solution, 100 µL of 10 mg/mL insulin solution was dropped, and the solution was stirred by homogenizer (15000 rpm, 5 minutes). After stirring, the solution was added to 20 mL of dioxane. The solvent was evaporated, and the solution was concentrated to about 2 mL, and the particulate dispersion solution was added to dioxane (6 mL) containing 500 mg of Pluronic (registered trademark of BASF) F68. The solution was freeze-dried, and a portion of 8 lots was dispersed in 80 mL of purified water. By ultrafiltration apparatus equipped with a filter membrane of fractional molecular weight of 100,000, the solution was concentrated to 10 mL, and 50 mL of purified water was added, and the solution was concentrated again to 5 mL. After repeating one more concentration operation, purified water was added to make up 40 mL. By adding 40 mL of 60 mg/mL Pluronic (registered trademark of BASF) F68, the solution was freeze-dried. In the freeze-dried powder sample, water was added to disperse again. The particle size of fine particles was measured by dynamic light scatter method by using apparatus Zetasizer 3000HSA (manufactured by Malvern Instruments). The obtained data was analyzed by CONTIN method and histogram method, and the particle size was calculated. To determine the insulin enclosing efficacy, the particulate sample was hydrolyzed for 22 hours in HC1, 105 °C, the amino acid was analyzed, and the protein mass was measured.

### <Results>

A total amount of 278.2 mg of particles in particle size of 300 nm was obtained. The insulin enclosing efficacy by amino acid analysis was 25%.

### Example 10

### Manufacture of fine particles

10 mg of polyvinyl pyrrolidone-poly (lactic acid), which is an amphiphilic polymer, (molecular weight of polyvinyl pyrrolidone is 1,700 and molecular weight of poly (lactic acid) is 2,200) was dissolved in 2 mL of ethyl acetate, and a polymer solution was prepared. In this polymer solution, 100 µL of 10 mg/mL insulin solution was dropped, and the solution was stirred for several seconds. The solution was added to 10 times in volume of dioxane. The solvent was evaporated, and the solution was concentrated to about 2 mL, and the particulate dispersion solution was added to dioxane (20 mL) containing 500 mg of Pluronic (registered trademark of BASF) F68. The solution was freeze-dried, and 2 mL of water was added, and the particles were dispersed again. The particle size of fine particles was measured by dynamic light scatter method by using apparatus Zetasizer 3000HSA (manufactured by Malvern Instruments). The obtained data was analyzed by CONTIN method and histogram method, and the particle size was calculated.

### <Results>

Sedimentation was noted after re-dispersion, but when the particle size was measured after removing the sediment by centrifugal separation, particles of 90 to 130 nm were formed.

### Example 11

### Manufacture of particles

10 mg of polyethylene glycol-poly (epsilon-caprolactone), which is an amphiphilic polymer, (molecular weight of polyethylene glycol is 5,000 and molecular weight of poly (epsilon-caprolactone) is 10,000) was dissolved in 2 mL of ethyl acetate, and a polymer solution was prepared. In this polymer solution, 100 µL of 10 mg/mL insulin solution was dropped, and the solution was stirred by homogenizer (15000 rpm, 5 minutes). After stirring, the solution was added to 20 mL of dioxane. The solvent was evaporated, and the solution was concentrated to about 2 mL, and the particulate dispersion solution was added to dioxane (8 mL) each containing various concentrations of Cremophor (registered trademark of BASF) EL (polyoxyethylene cured castor oil). The solution was freeze-dried, and 10 mL of purified water was added, and the particles were dispersed by ultrasonic process. The particle size of fine particles was measured by dynamic light scatter method by using apparatus Zetasizer 3000HSA (manufactured by Malvern Instruments). The obtained data was analyzed by CONTIN method and histogram method, and the particle size was calculated.

### <Results>

The particle size after dispersion in purified water decreased along with concentration of Cremophor (registered trademark of BASF) EL, and was 61 nm at concentration of 1000 mg/mL of Cremophor (registered trademark of BASF) EL (Fig. 6).

### Example 12

### Drug effect durability of insulin contained in particles

A particulate preparation enclosing 15 µg (WAKO) of insulin prepared in a method of example 9 was dispersed in 100 µL of normal saline (Otsuka), and was administered to 7-week-old male BALB/c mice (weighing 19 to 24 g) after fasting for 12 hours, under the dorsal skin by using 29G injection needle (Terumo) (in the control group, 100 µL of normal saline (Otsuka) dissolving 15 µg of insulin was used). After administration, animals were kept fasted in individual cages, and blood samples collected from the caudal artery before and after administration were tested by biochemical automatic analyzer (Fuji Drychem 3500V of Fuji Film), by using measuring slide (GLU-WIII of Fuji Film), and changes of glucose concentration were measured at time intervals, and the average of serum glucose concentration changes of two animals each was calculated.

### <Results>

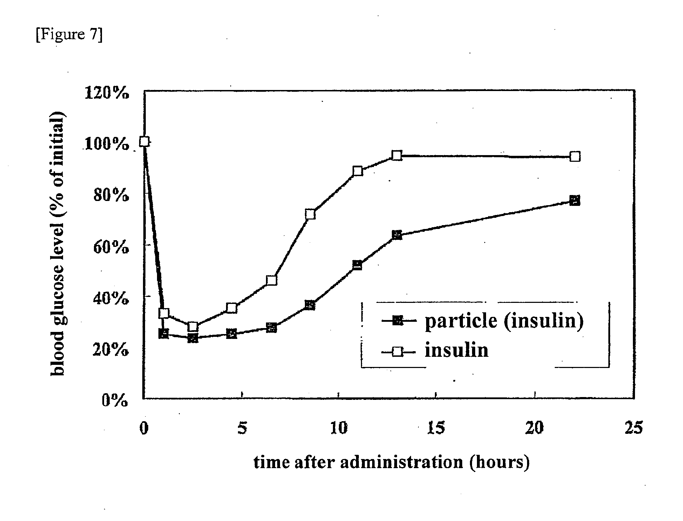

In the mice administered insulin only, the serum glucose concentration recovered to 80% of the level before administration in 10 hours, but in the mice administered particulate preparation enclosing insulin, decline of serum glucose concentration continued, maintaining at about 40% in 10 hours, and about 70% before administration in 23 hours, and the drug effect durability due to sustained release property of the particulate preparation was recognized (Fig. 7).

### INDUSTRIAL APPLICABILITY

A fine particle of the inventions realizes stability enhancement in body and improvement of behavior in body of protein, peptide drug, nucleic acid and other medicine of hydrophilic property and large molecular weight. Oral or enteric administration of bioactive protein is possible, and as compared with the conventional administration method by injection, easy and less painful medication is realized for patients. In administration by injection, too, sustained release by hypodermic administration, or extension of circulating time in blood by administration in blood are possible, and the number of times of injection can be decreased.

The medicinal preparation including a fine particle of the inventions is expected to maintain the pharmacological effect for a long period. The medicinal preparation including a fine particle of the inventions is controllable in drug release characteristic by selection of matrix forming materials.

## Claims

1. A fine particle comprising an amphiphilic polymer, further comprising an inner nucleus of hydrophilic segment of amphiphilic polymer, and a hydrophobic outer layer of hydrophobic segment of amphiphilic polymer, and having a surface modifier bonded to the hydrophobic outer layer.

2. The fine particle according to claim 1, wherein the surface modifier is a hydrophilic polymer.

3. The fine particle according to claim 2, wherein the hydrophilic polymer is any one selected from the group consisting of polyethylene glycol, polyvinyl pyrrolidone, polyvinyl alcohol, polyethylene imine, polyacrylic acid, polymethacrylic acid, poly-1,3-dioxolane, 2-methacryloyl oxyethyl phosphoryl choline polymer, poly-1,3,6-trioxane, polyamino acid, protein, and polysaccharides.

4. The fine particle according to claim 2, wherein the hydrophilic polymer is an analog of any one selected from the group consisting of polyethylene glycol, polyvinyl pyrrolidone, polyvinyl alcohol, polyethylene imine, polyacrylic acid, polymethacrylic acid, poly-1,3-dioxolane, 2-methacryloyl oxyethyl phosphoryl choline polymer, poly-1,3,6-trioxane, polyamino acid, protein, and polysaccharides.

5. The fine particle according to claim 3 or 4, wherein the polyamino acid is an analog of polyaspartic acid or polyglutamic acid.

6. The fine particles according to claim 3 or 4, wherein the protein is gelatin, casein, or albumin.

7. The fine particle according to claim 3 or 4, wherein the polysaccaride is any one selected from the group consisting of cellulose, chitin, chitosan, gellan gum, arginic acid, hyaluronic acid, pullulan, and dextran.

8. The fine particle according to claim 3 or 4, wherein the polysaccaride is an analog of any one selected from the group consisting of cellulose, chitin, chitosan, gellan gum, arginic acid, hyaluronic acid, pullulan, and dextran.

9. The fine particle according to claim 1, wherein the surface modifier is an amphiphilic compound.

10. The fine particle according to claim 9, wherein the amphiphilic compound is a surfactant.

11. The fine particle according to claim 10, wherein the surfactant is any one selected from the group consisting of polyoxy ethylene-polypropylene glycol copolymer, sucrose fatty acid ester, polyethylene glycol fatty acid ester, polyethylene sorbitan mono-fatty acid ester, polyoxyethylene sorbitan di-fatty acid ester, polyoxy ethylene glycerin mono-fatty acid ester, polyoxy ethylene glycerin di-fatty acid ester, polyoxy ethylene castor oil, polyoxy ethylene cured castor oil, alkyl sulfate and lecithin.

12. The fine particle according to claim 1, wherein the hydrophilic segment of amphiphilic polymer comprising any one selected from the group consisting of polyethylene glycol, polyvinyl pyrrolidone, polyvinyl alcohol, polyethylene imine, polyacrylic acid, polymethacrylic acid, poly-1,3-dioxolane, 2-methacryloyl oxyethyl phosphoryl choline polymer, poly-1,3,6-trioxane, polyamino acid, and polysaccharides.

13. The fine particle according to claim 1, wherein the hydrophilic segment of amphiphilic polymer comprising an analog of any one selected from the group consisting of polyethylene glycol, polyvinyl pyrrolidone, polyvinyl alcohol, polyethylene imine, polyacrylic acid, polymethacrylic acid, poly-1,3-dioxolane, 2-methacryloyl oxyethyl phosphoryl choline polymer, poly-1,3,6-trioxane, polyamino acid, and polysaccharides.

14. The fine particle according to claim 1, wherein the hydrophobic segment of amphiphilic polymer comprising any one selected from the group consisting of aliphatic polyester, polyorthoester, polycyano-acrylic ester, polyalkylene glycol, polyurethane, polysiloxane, polyamino acid, and poly acid anhydride.

15. The fine particle according to claim 1, wherein the hydrophobic segment of amphiphilic polymer comprising an analog of any one selected from the group consisting of aliphatic polyester, polyorthoester, polycyano-acrylic ester, polyalkylene glycol, polyurethane, polysiloxane, polyamino acid, and poly acid anhydride.

16. The fine particle according to claim 14 or 15, wherein the aliphatic polyester is poly (epsilon-caprolactone), polylactic acid, or polyglycolic acid.

17. The fine particle according to claim 1, wherein the hydrophobic segment of amphiphilic polymer is a biodegradable polymer.

18. The fine particle according to claim 1, wherein the amphiphilic polymer is a copolymer having any structure of block structure, graft structure, or branch structure.

19. The fine particle according to claim 1, wherein the mean particle size is 50 to 1000 nm.

20. A fine particle comprising an inner nucleus of hydrophilic segment of amphiphilic polymer, and a hydrophobic outer layer of hydrophobic segment of amphiphilic polymer, wherein:
(A) the hydrophobic segment of amphiphilic polymer comprising any one selected from the group consisting of aliphatic polyester, polyorthoester, polycyano-acrylic ester, polyalkylene glycol, polyurethane, polysiloxane, polyamino acid, and polyacid anhydride, and
(B) the hydrophilic segment of amphiphilic polymer comprising any one selected from the group consisting of polyethylene glycol, polyvinyl pyrrolidone, polyvinyl alcohol, polyethylene imine, polyacrylic acid, polymethacrylic acid, poly-1,3-dioxolane, 2-methacryloyl oxyethyl phosphoryl choline polymer, poly-1,3,6-trioxane, and polysaccharides.

21. The fine particle according to claim 20, wherein the mean particle size is 25 nm to 5 µm.

22. The fine particle according to claim 1 or 20, wherein a drug is contained in the inner nucleus of hydrophilic segment of amphiphilic polymer.

23. A pharmaceutical preparation comprising the fine particle according to claim 1 or 20.
